# EUROPEAN PATENT APPLICATION

(11) **EP 2 997 894 A1**
(43) Date of publication of application: **23.03.2016**
(21) Application number: 15185384.3
(22) Date of filing: 16.09.2015
(51) Int. Cl.: A61B 5/097, A61M 39/10, A61B 5/083, G01N 33/497

(54) **GAS SAMPLING CONNECTOR**

(30) Priority: 17.09.2014 US 201414488310
(71) Applicant: Oridion Medical 1987 Ltd., Jerusalem 91450 (IL)
(72) Inventor: WEISS, Nir, 7662085 Rehovot (IL); PEER, Roni, 5629003 Yehud (IL); ERLICH, Noam, 7682900 Naan (IL); PERY, Nimrod, 5222533 Ramat-Gan (IL)
(74) Representative: Awapatent A/S

(57) **Abstract**

A connector includes a memory configured to store data. The connector further includes a first portion configured to connect to a ground connection in a gas sampling monitor. The connector further includes a second portion configured to connect to a positive connection in the gas sampling monitor. The second portion is configured to transfer data to and from the memory. The connector further includes an end configured to connect with a sampling tube, which is configured to transmit gas to the monitor.

## Description

### TECHNICAL FIELD

The present disclosure relates generally to gas sampling tubes, and more particularly to a smart connector for gas sampling.

### BACKGROUND

Present gas sampling lines, also referred to as filter-lines, gas lines, sampling tubes, and other similar names transfer gas to and from devices such as end-tidal carbon dioxide (EtCO2) monitors that measure gas from a patient. For example, many gas measuring devices connect to a patient using an external oral cannula, an external nasal cannula, an external oral/nasal cannula, or an external connection from a endotracheal tube. These gas sampling lines are unintelligent and do not do anything more than transfer gas to and from a patient's nose and mouth.

### SUMMARY

According to the present disclosure, disadvantages and problems associated with previous gas sampling tubes may be reduced or eliminated.

In certain embodiments, a connector includes a memory configured to store data. The connector further includes a first portion configured to connect to a ground connection in a gas sampling monitor. The connector further includes a second portion configured to connect to a positive connection in the gas sampling monitor. The second portion is configured to transfer data to and from the memory. The connector further includes an end configured to connect to a sampling tube, which is configured to transmit gas to the monitor.

Certain embodiments of the present disclosure may provide one or more technical advantages. In conventional gas sampling systems, sampling line connectors are unintelligent devices. It is not possible to distinguish between sampling lines and it is not possible to track how long sampling lines have been used, which may lead to patient contamination. In certain embodiments of this disclosure, an improved smart connector device is designed to be recognized, authenticated, and tracked. In certain embodiments, the novel design includes a memory incorporated in the smart connector. In certain embodiments, this design ensures that a connection is present and includes technological advantages over simple conventional sampling lines. In these embodiments, the clinician may monitor several features of the sampling lines such as a unique ID and lot number, a recommended duration of use, a recommended number of connections to filter-lines, a recommended life-time after first connection, a date and time of first connection, a duration of operation while connected, a number of connections made to the connector.

Certain embodiments of the present disclosure may include some, all, or none of the above advantages. One or more other technical advantages may be readily apparent to those skilled in the art from the figures, descriptions, and claims included herein. Moreover, while specific advantages have been enumerated above, various embodiments may include all, some, or none of the enumerated advantages.

### BRIEF DESCRIPTION OF THE DRAWINGS

For a more complete understanding of the present disclosure and its features and advantages, reference is now made to the following description, taken in conjunction with the accompanying drawings, in which:
FIGURE 1 illustrates an example gas sampling system that includes a smart connector, according to certain embodiments of the present disclosure;
FIGURE 2 illustrates details of an example smart connector in FIGURE 1, according to certain embodiments of the present disclosure;
FIGURE 3A illustrates one example perspective view of a smart connector, according to certain embodiments of the present disclosure;
FIGURE 3B illustrates details of the smart connector of FIGURE 3B, according to certain embodiments of the present disclosure;
FIGURE 4 illustrates details of another example smart connector, according to certain embodiments of the present disclosure; and
FIGURE 5 illustrates an example method for implementing a gas sampling device, according to certain embodiments of the present disclosure;
FIGURE 6 illustrates details of another example smart connector with a contact face, according to certain embodiments of the present disclosure; and
FIGURE 7 illustrates details of another example smart connector with contact in a reader, according to certain embodiments of the present disclosure.

### DESCRIPTION OF EXAMPLE EMBODIMENTS

A capnograph measures air exhaled by patient through a tube. This tube may also be referred to as a gas sampling line or a gas sampling device as it is used in this disclosure. One end of the gas sampling device is connected to the patient through an external mechanism such as a mask, cannula, endotracheal tube, or an external connection to a respirator and/or ventilator. The other end of the gas sampling device is connected to a gas sampling monitor, in this particular example a capnograph, with a connector. The complete gas sampling tube plus gas sampling monitor may be referred to as a gas sampling system.

Current gas sampling systems are limited because sampling line connectors are unintelligent devices. These devices lack the technological sophistication that clinicians may expect of other sensors, such as pulse oximetry sensors. This is because gas sampling line connectors have no way to create a connection to drive power to the connector. Thus, conventional gas sampling systems require clinicians to manually associate gas sampling lines with patients. Further, it is not possible for clinicians to distinguish between gas sampling lines and it is not possible to track how long sampling lines have been used, which may lead to patient contamination.

In certain embodiments of this disclosure, an improved smart connector device is incorporated in gas sampling lines and is designed to be recognized, authenticated, and tracked. In certain embodiments, the novel design includes a memory incorporated in the smart connector. In certain embodiments, the design ensures that a connection is present and includes technological advantages over simple conventional sampling lines. In these embodiments, the clinician may monitor several features of the sampling lines such as a unique ID and lot number, a recommended duration of use, a recommended number of connections to filter-lines, a recommended life-time after first connection, a date and time of first connection, a duration of operation while connected, a number of connections made to the connector.

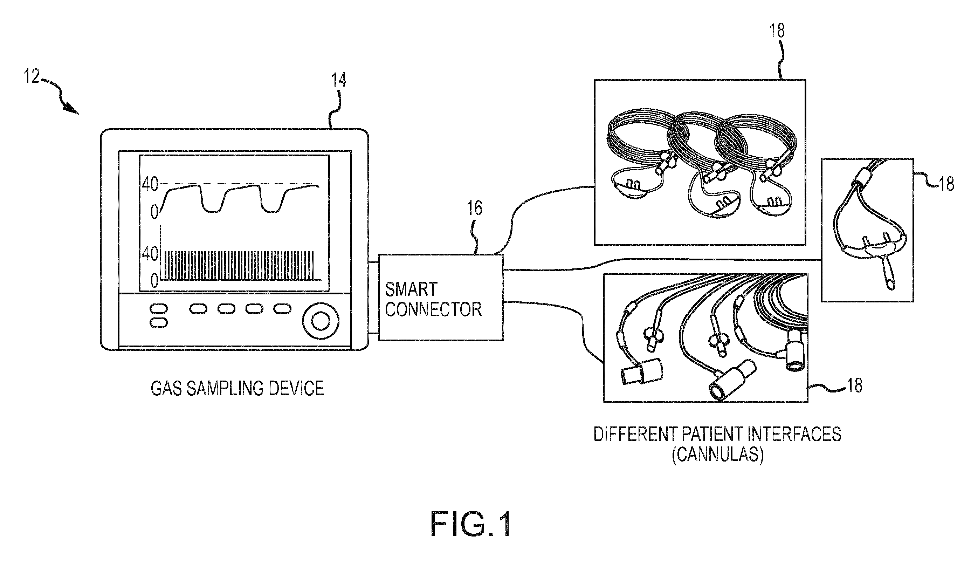

According to certain embodiments of the present disclosure, the gas sampling monitor is connected to the smart connector. The gas sampling monitor is configured to receive transmitted gases from the sampling lines that include the smart connector and may be configured to read and write data from the smart connector. The gas sampling monitor may analyze the transmitted gas to determine where one or more characteristics of the transmitted gases and to generate data for display indicative of the characteristics of the transmitted gases. In this example, the characteristics may include at least an EtCO2 measurement. Thus, the gas sampling system allows clinicians to monitor both the EtCO2 of the patient and ensure that the gas sampling lines are recognized, authenticated, and tracked. Additional details of example embodiments of the disclosure are described below with reference to FIGURES 1-4.

FIGURE 1 illustrates an example gas sampling system 12. In the illustrated embodiment, gas sampling system 12 includes a gas sampling device 14, a smart connector 16, and tubes connected to cannulas 18. Each of smart connector 16 and tubes and cannulas 18 may be referred to as portions of gas sampling lines, gas sampling tubes. However, in certain other embodiments, each of these portions may be arranged and different configurations. Thus, the present disclosure contemplates any suitable arrangement of these portions of gas sampling system 12.

Gas sampling device 14 may refer to any suitable device operable to analyze gas. In certain embodiments, the gas sampling device 14 may include a capnograph. In certain other embodiments, the gas sampling device 14 may include a multiparameter monitoring device that monitors gas along with other patient parameters such as oxygen saturation. Transmission of sampled gas from the gas sampling device to the gas sampling device 14 allows a clinician to monitor certain characteristics of a sampled gas, such as EtCO2.

Tube and cannulas 18 may refer to any suitable tube and/or cannula for transmitting gas and connecting to a patient as an interface to send and receive gas from the patient. Tube and cannula 18 may be configured to be connected to an end of smart connector 16. For example, the tube and cannula 18 may refer to a plastic tube that attached, connected, or inserted in an end of smart connector 16 and tube and cannula 18 may include an inner wall where gas is transmitted from a patient to a gas sampling monitor. Tube and cannula 18 may be a flexible tube, in certain embodiments. In certain other embodiments, tube and cannula 18 or may be a rigid tube. In certain embodiments, tube and cannula 18 may include a filter. The filter may include any device that filters moisture, fluid, contaminants, or any other similar substance. For example the filter may include a hydrophilic wick. In certain other embodiments, the filter may include a multi-use filter, also referred to as a long term filter. In certain embodiments, the filter may be incorporated in one or more portions of smart connector 16.

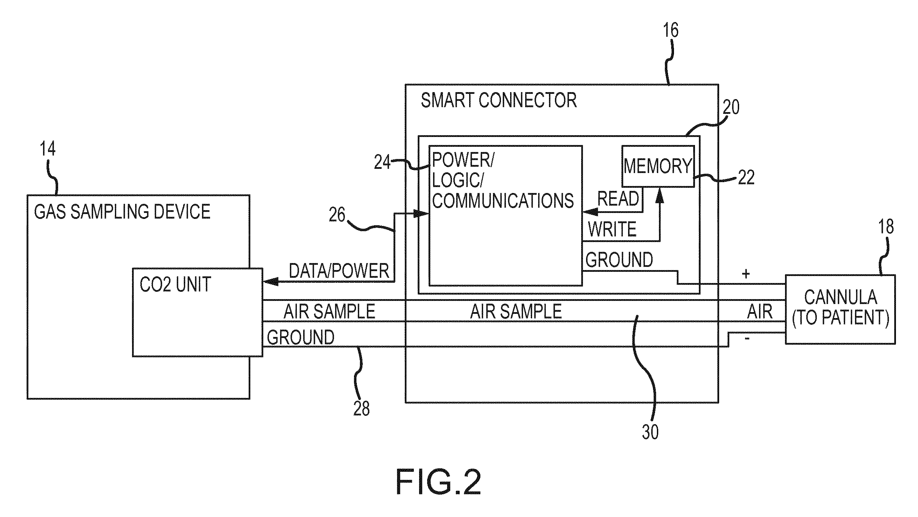

Smart connector 16 may refer to any suitable connector for connecting gas sampling device 14 to a sampling line. For example, smart connector 16 may refer to a luer connector or a modified luer connector configured to satisfy an industry standard. Additional details of smart connector 16, according to certain embodiments, are described below with greater detail with reference to FIGURES 2-4.

FIGURE 2 illustrates details of an example smart connector 16 in FIGURE 1, according to certain embodiments of the present disclosure. Smart connector 16 may be substantially similar to smart connector 16 of FIGURE 1.

Smart connector 16 includes at least one memory 22 and at least one power/logic communication interface (I/F) 24. In certain other embodiments, smart connector may include a processor, an output device, and an input device, which are discussed in further detail below. Although this particular implementation of smart connector 16 is illustrated and primarily described, the present disclosure contemplates any suitable implementation of smart connector according to particular needs.

Memory 22 may include any suitable device operable for storing data and instructions. Memory 22 may include, for example, a magnetic disk, flash memory, optical disk, or other suitable data storage device. Memory 22 may include any computer memory (for example, Random Access Memory (RAM) or Read Only Memory (ROM)), mass storage media (for example, a hard disk), removable storage media (for example, a Compact Disk (CD) or a Digital Video Disk (DVD)), database and/or network storage (for example, a server). Memory 22 may comprise any other computer-readable tangible medium, or a combination of any of the preceding.

I/F 24 may include any suitable device operable to receive input smart connector 16, send output from smart connector 16, perform suitable processing of the input or output or both, communicate to other devices, or any combination of the preceding. I/F 24 may include appropriate hardware (for example, a modem, network interface card, etc.) and software, including protocol conversion and data processing capabilities, to communicate through a Serial Network, LAN, WAN, or other communication system that allows smart connector 16 to communicate data from memory 22 to other devices. I/F 24 may include one or more ports, conversion software, or a combination of any of the preceding.

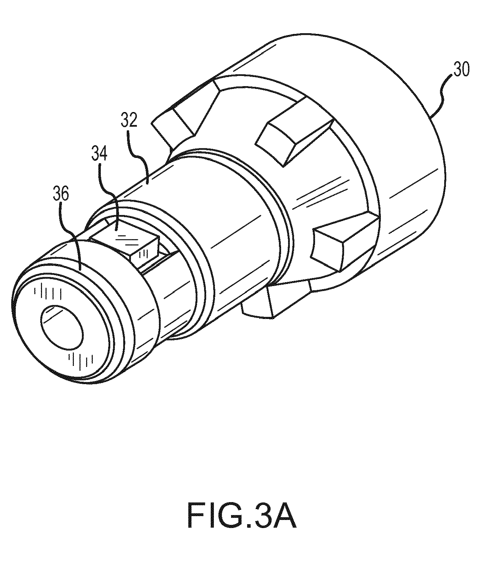

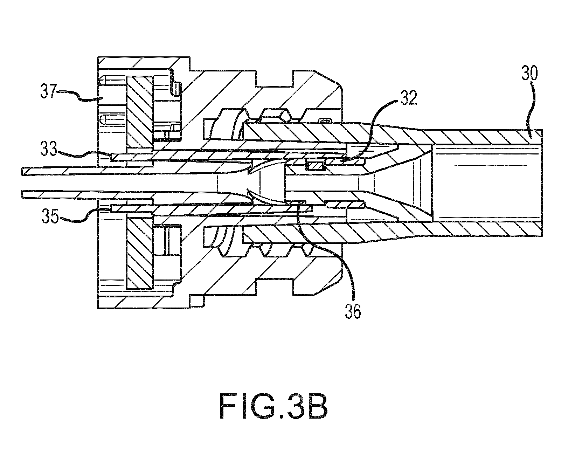

Smart connector 16 may include a processor, input device, and output device. The processor may include any suitable device operable to execute instructions and manipulate data to perform operations for smart connector 16. The processor may include, for example, any type of central processing unit (CPU). The output device may include any suitable device operable for displaying information to a user. The output device may include, for example, a video display, a printer, a plotter, or other suitable output device. In certain embodiments, the output device may reformat data in any suitable format to be transmitted to other systems. The input device may include any suitable device operable to input, select, and/or manipulate various data and information. The input device may include, for example, a keyboard, mouse, graphics tablet, joystick, light pen, microphone, scanner, or other suitable input device. Modifications, additions, or omissions may be made smart connector 16 without departing from the scope of the disclosure. The components of smart connector 16 may be integrated or separated. Moreover, the operations of smart connector 16 may be performed by more, fewer, or other components. Additionally, operations of smart connector 16 may be performed using any suitable logic. As used in this document, "each" refers to each member of a set or each member of a subset of a set. Further details of an example smart connector 16 and the operations of mobile patient monitor interface 214 are provided below with reference to FIGURE 3.

In one example embodiment of operation, smart connector 16 is inserted to the CO2 unit, which may also be referred to as a CO2 module, which has a receptacle in gas sampling device 14. In the example embodiment, a connection in smart connector 16 is engaged with gas sampling device 14 and an electrical circuit is closed by connecting a data/power communication layer 26 and a ground connection layer 28 with gas sampling device 14, which enables communication with memory 22 on smart connector 16. In the example embodiment, data may be transferred a data/power communication layer 26 implemented by I/F 24 of smart connector 16. This data may contain identification information of smart connector 16, as described in more detail below. If the information the CO2 unit receives is indicative of an authentic connector, the sampling begins and gas sampling device 14 may begin operating and receiving or transmitting gas as indicated by reference number 30 in FIGURE 2.

In another example embodiment of operation, memory 22 may be configured to save operation data. Such data may include operation time, number of connections, and other similar data. In these embodiments, if smart connector 16 is disconnected from gas sampling device 14, an electrical circuit may be opened and, therefore, ommunication between smart connector 16 and gas sampling 14 may no longer be available and data is no longer stored on or read from smart connector 16. Other examples of data that may be stored on memory 22 include: consumable classes such as Adult, Pediatric, Neonate; consumable types such as intubated, non-intubated; default monitor settings according to class and/or type; manufacturer such as Covidien, OEM, etc.; life cycle time of consumable according to type; lot, batch, revision, manufactured date; number of allowed insertions; total number of insertions; material of consumable; total usage time of consumable; occlusion time of the consumable; patient information: name, age, type (pediatric, adult, neonate); monitor settings and type; usage records that can follow the patient between areas of care; medical staff comments or remarks during treatment, indications of treatment; measurement data and information of extreme patient status; malfunctions or errors during measurements; known disease or illness related to the device or consumable; intubated or not; if changed during the consumable usage - time and date; area(s) of care in which was in use; or other interface to read/pass the data to a host computer/software.

Therefore, certain embodiments of this disclosure describe a smart connector 16 that is designed to be recognized, authenticated, and tracked by clinicians and/or gas sampling device 14. In certain embodiments, the novel design includes memory 22 incorporated in the smart connector. In certain embodiments, this design ensures that a connection is present and includes technological advantages over simple conventional sampling lines. In these embodiments, memory 22 may store and the clinician may monitor several features of the sampling lines such as a unique ID and lot number, a recommended duration of use, a recommended number of connections to filter-lines, a recommended life-time after first connection, a date and time of first connection, a duration of operation while connected, a number of connections made to the connector.

FIGURE 3A illustrates one example perspective view of a smart connector 30, according to certain embodiments of the present disclosure. Smart connector 30 may be substantially similar to smart connector 16 of FIGURES 1 and 2. As described above and throughout this disclosure, in order to maintain the best accuracy of CO2 measurements, new gas sampling lines are designed to be recognized, identified, authenticated, and tracked. Smart connector 30 includes an enhancement of the basic identification process using a directional electrical connection that utilizes a first portion and a second portion. In the illustrated embodiment, the first portion includes a first ring 32 and the second portion includes a second ring 36. Rings 32 and 36, which are the first portion and second portion in this example embodiment, authenticate and implement technical features in smart connector 30 using a memory 34.

In one example embodiment of operation described with reference to FIGURE 3B, when smart connector 16 is inserted in a receptacle 37, an electrical bridge is closed by connecting a positive layer incorporated in ring 32 with a positive layer 33 in receptacle 37 and connecting a ground layer incorporated in ring 36 with a ground layer 35, thus allowing connectivity between the module and the connector. In certain other embodiments, closing the electrical bridge may also be referred to as engaging the smart connector with the gas sampling device, which enables identification, authorization, tracking, and communication with memory 22 on smart connector 16. In certain embodiments, the positive layer may include a data/power communication layer to communicate data to and from smart connector 30. The connection process may also be referred to as an identification process. This identification may ensure that a particular connector is present and facilitates data transfer to and from smart connector 16. Thus, smart connector 30 with two contact rings 32 and 36 implements a data and electrical connection that is directional in a manner that the positive and ground electric leads are connected in the same manner, regardless of orientation. Although illustrated as cylindrical rings 32 and 36, the first portion and second portion of smart connector 30 may be implemented using any suitable shape or other similar mechanism such as one or more metal cones.

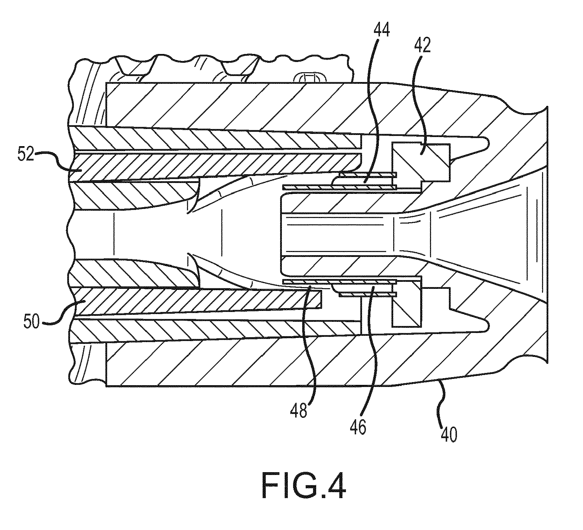

FIGURE 4 illustrates details of another example smart connector, according to certain embodiments of the present disclosure. The smart connector in FIGURE 4 includes a memory 44 that may be similar to memory 34 in FIGURE 3A and memory 22 in FIGURE 2. In contrast to FIGURE 3A, the smart connector in FIGURE 4 includes a first portion and a second portion in the form of parallel contact rings 44 and 48, as opposed to the two contact rings 32 and 36 in sequence in FIGURE 3A. In one example embodiment of operation described with reference to FIGURE 4, when the smart connector is inserted in a receptacle, an electrical bridge is closed by connecting a positive layer incorporated in ring 44 with a positive layer 52 and connecting a ground layer incorporated in ring 48 with a ground layer 50, thus allowing connectivity between the CO2 module and the smart connector. In certain embodiments, the positive layer may include a data/power communication layer to communicate data to and from the smart connector in FIGURE 4.

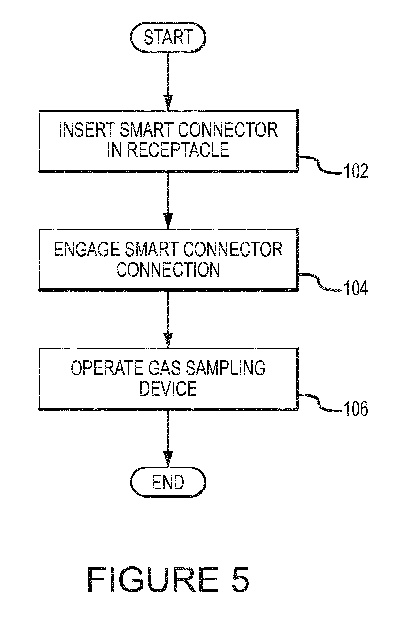

FIGURE 5 illustrates an example method 100 for implementing a gas sampling device, according to certain embodiments of the present disclosure. Method 100 begins at step 102 where a smart connector is inserted into a receptacle. For example, the smart connector may be inserted into a receptacle, CO2 unit or module of a gas sampling device, such as a capnograph as described above with reference to FIGURES 1 and 2.

At step 104, as the smart connector is inserted in the receptacle, the smart connector is engaged with the gas sampling device and an electrical bridge is closed by connecting a first portion of the smart connector and a second portion of the smart connector with features in the receptacle. For example, a positive layer incorporated in the first portion in the form of a ring may be connected with a positive layer in the receptacle and a ground layer incorporated in the second portion in the form of a second ring may be connected with a ground layer in the receptacle, thus allowing connectivity between the CO2 unit and the smart connector. This process enables identification, authorization, tracking, and communication with a memory in smart connector. For example, in certain embodiments, the positive layer may include a data/power communication layer to communicate data to and from the smart connector. Thus, the smart connector implements a data and electrical connection and triggers the gas sampling device to begin operation.

Next, at step 106, the gas sampling device is operated based on the engagement described above and gas is transmitted to the gas sampling device to a gas sampling monitor. In certain embodiments, the gas sampling device may include a capnograph. In certain other embodiments, the gas sampling device may include a multiparameter monitoring device that monitors gas along with other patient parameters such as oxygen saturation. Transmission of sampled gas from the gas sampling device allows a clinician to monitor certain characteristics of a sampled gas, such as EtCO2.

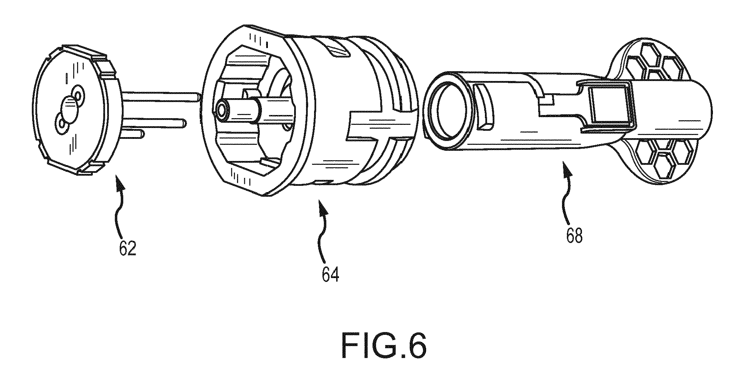

FIGURE 6 illustrates details of another example smart connector, according to certain embodiments of the present disclosure. The smart connector in FIGURE 6 includes a memory 68 that may be similar to memory 34 in FIGURE 3A and memory 22 in FIGURE 2. In contrast to other embodiments, the smart connector in FIGURE 6 includes a contact face 64 that connects with positive and ground pins 62, as opposed to one or more contact rings, such as contact rings 32 and 36 in sequence in FIGURE 3A. In one example embodiment of operation described with reference to FIGURE 6, when the smart connector is inserted in a receptacle, an electrical bridge is closed by connecting a positive layer incorporated in 64 with a positive layer in pins 62. In an alternate embodiment, a first contact may be made to an internal ring such as contact rings 32 and 36 in sequence in FIGURE 3A and a second contact on face 64 may be made using a third spring-type pin incorporated in pins 62. In another alternate embodiment, the smart connector may include one or more pins, and the smart connector or a smart connector reader may implement a split ring where the split ring includes, for example, three unique sections that enable two-contacts, using electrical switch and may have electrical insulation between sections. In certain embodiments, the positive layer may include a data/power communication layer to communicate data to and from the smart connector in FIGURE 6.

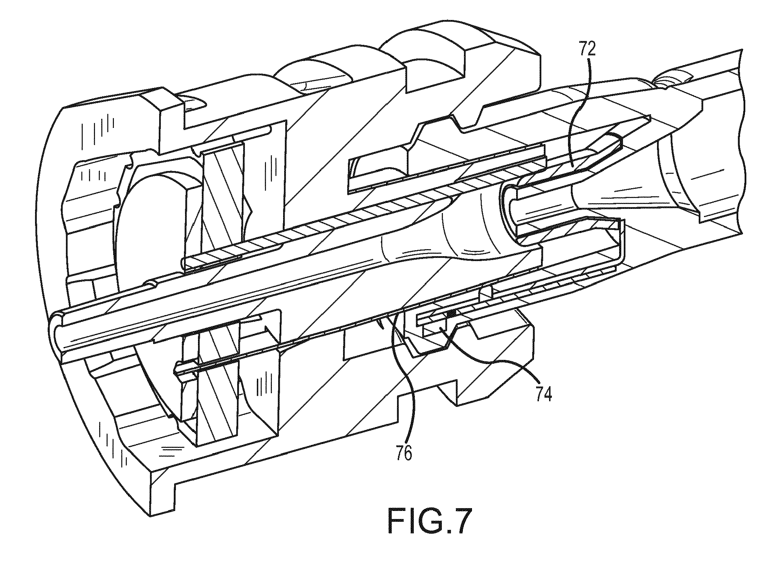

FIGURE 7 illustrates details of another example smart connector, according to certain embodiments of the present disclosure. The smart connector in FIGURE 7 includes a memory 74 that may be similar to memory 34 in FIGURE 3A and memory 22 in FIGURE 2. In contrast to other embodiments, the smart connector in FIGURE 7 achieves contact using two metal cones, one placed on the smart connector as indicated by reference number 72 and a second placed on the reader (also referred to as the receptacle) as indicated by reference number 76. In one example embodiment of operation described with reference to FIGURE 7, when the smart connector is inserted in a receptacle, an electrical bridge is closed by connecting a positive layer incorporated in 72 and a ground layer incorporated in 76. In certain embodiments, the positive layer may include a data/power communication layer to communicate data to and from the smart connector in FIGURE 7.

Although this disclosure has been described in terms of certain embodiments, alterations and permutations of the embodiments will be apparent to those skilled in the art. Accordingly, the above description of the embodiments does not constrain this disclosure. Other changes, substitutions, and alterations are possible without departing from the spirit and scope of this disclosure, as defined by the following claims.

## Claims

1. A connector, comprising:
a memory configured to store data;
a first portion configured to connect to a ground connection in a gas sampling device;
a second portion configured to connect to a positive connection in the gas sampling device, the second portion further configured to transfer data to and from the memory; and
an end configured to be connected with a sampling tube configured to transmit gas to the gas sampling device.

2. The connector of claim 1, wherein the connector comprises a modified luer connector.

3. The connector of claim 1, wherein the first portion comprises a filter.

4. The connector of claim 1, wherein the first portion comprises a ring.

5. The connector of claim 1, wherein the memory stores a unique ID and lot number.

6. The connector of claim 1, wherein the memory stores a recommended duration of use.

7. The connector of claim 1, wherein the memory stores a recommended number of connections.

8. The connector of claim 1, wherein the memory stores a recommended life-time after first connection.

9. The connector of claim 1, wherein the memory stores a duration of operation while connected.

10. The connector of claim 1, wherein the memory stores a date and time of first connection.

11. The connector of claim 1, wherein the memory stores a number of connections made to the connector.

12. A method, comprising:
inserting a connector into a gas sampling device, the connector comprising:
a memory configured to store data;
a first portion configured to connect to a ground connection in a gas sampling device;
a second portion configured to connect to a positive connection in the gas sampling device, the second portion further configured to transfer data to and from the memory; and
an end configured to be connected with a sampling tube configured to transmit gas to the gas sampling device; and
engaging the connector with the gas sampling device and closing an electrical bridge by connecting the first portion and the second portion of the smart connector with the gas sampling device.

13. The method of claim 12, wherein the connector comprises a modified luer connector.

14. The method of claim 12, wherein the first portion comprises a filter; or wherein the first portion comprises a ring.

15. The method of claim 12, wherein the memory stores a unique ID and lot number; or
wherein the memory stores a recommended duration of use; or
wherein the memory stores a recommended number of connections; or
wherein the memory stores a recommended life-time after first connection; or wherein the memory stores a duration of operation while connected.
